# EUROPEAN PATENT APPLICATION

(11) **EP 3 305 368 A1**
(43) Date of publication of application: **11.04.2018**
(21) Application number: 16192828.8
(22) Date of filing: 07.10.2016
(51) Int. Cl.: A61N 5/10

(54) **PARTICLE THERAPY APPARATUS COMPRISING AN MRI**

(71) Applicant: Ion Beam Applications, 1348 Louvain-la-Neuve (BE)
(72) Inventor: Forton, Eric, 1348 Louvain-la-Neuve (BE)
(74) Representative: De Groote, Christophe

(57) **Abstract**

A particle therapy apparatus (1) for irradiating a target (5) with a charged particle beam (2) and comprising a particle accelerator and an isocentric gantry (10) rotatable about an axis Y. The gantry (10) comprises a sequence of bending magnets to successively bend and finally direct the particle beam (2) towards the isocentre (20) of the gantry (10). A last bending magnet (15) of said sequence is configured to bend the particle beam (2) in a first plane P1 comprising the isocentre (20) and making a large angle with the axis Y. The gantry (10) also comprises a first scanning magnet (31) arranged upstream of the last bending magnet (15) and configured to scan the particle beam (2) over the target (5) in the first plane P1. The apparatus also comprises a magnetic resonance imaging (MRI) system having two main magnet units (41, 42) arranged on both sides of the isocentre (20) and configured to generate a main magnetic field (Bo) which is parallel to or coaxial with the axis Y. MRI imaging of the target (5) at the isocentre (20) can hence be achieved while or while not scanning the particle beam (2) over the target (5).

## Description

### Field of the invention

The invention relates to an apparatus for irradiating a target with a charged particle beam for therapy purposes and comprising a magnetic resonance imaging (MRI) system for imaging the target.

### Background of the invention

Such a particle therapy apparatus is known from patent publication numbers WO2010067287 and WO2015197475.

Such known medical apparatus comprises a conventional particle therapy apparatus for irradiating a target volume of a patient with a beam of energetic charged particles, and a magnetic resonance imaging (MRI) system arranged around the target volume for imaging the target in real time in the course of the irradiation of the target with the particle beam.

The particle therapy apparatus includes a conventional rotatable gantry which comprises a plurality of bending magnets to successively bend and finally direct the particle beam towards the isocentre of the gantry, where the target will be placed for treatment. The MRI system is arranged in such a way that the direction of its main magnetic field (Bo) is the same as the direction of the axis of rotation of the gantry, which is generally chosen to be a horizontal direction for the comfort of the patient who can then be placed horizontally in the particle therapy apparatus, as shown on Fig.1 and Fig.2 of WO2015197475.

As can be seen from Fig.1 WO2015197475, the gantry 156 comprises a last bending magnet 158 which bends the particle beam 154 in a plane comprising the axis of rotation R of the gantry and which directs the beam towards the target 124 in a direction perpendicular to the axis of rotation R.

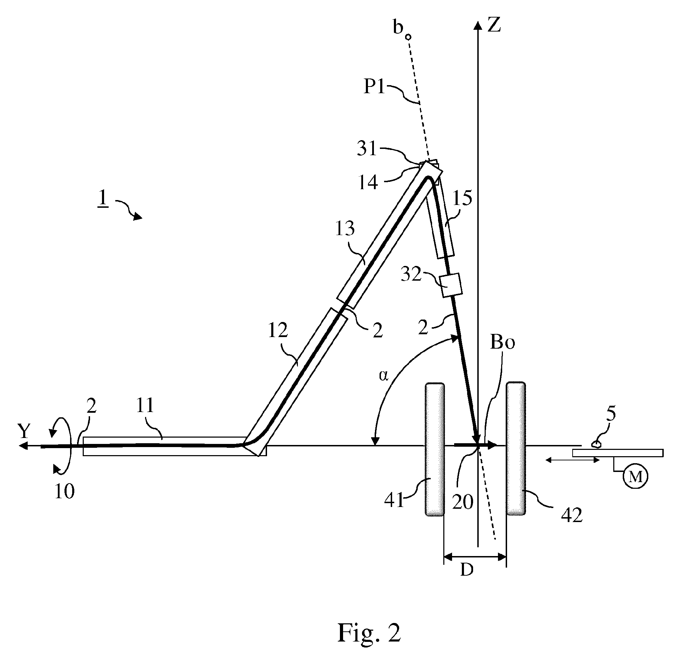

The apparatus also comprises scanning magnets to scan the particle beam across the target. These scanning magnets are arranged inside a low-field ring 166. As can be seen on Fig.2, this low-field ring is located downstream of the last bending magnet 158 of the gantry. Hence, these scanning magnets are arranged downstream of the last bending magnet of the gantry.

Such an apparatus presents the drawback that the fast changing magnetic field generated by the scanning magnets may negatively influence the main magnetic field (Bo) of the MRI system, which knowingly must be as constant as possible in order, among others, not to introduce artefacts in the acquired MRI images. A solution might be to increase the radial distance between the scanning magnets and the isocentre, but this in turn will increase the radius of the gantry and hence its bulk and cost. Such an arrangement also presents the drawback that the scanning magnets should be placed in the low-field ring, which may put constraints on other design parameters and hence impair on the design freedom for the apparatus.

### Summary of the invention

It is an object of the invention to address the problems of the state of the art particle therapy apparatus. It is more particularly an object of the invention to provide a scanning particle beam therapy apparatus comprising an MRI system and which is more compact than the known apparatus.

The invention is defined by the independent claims. The dependent claims define advantageous embodiments.

According to the invention, there is provided a particle therapy apparatus for irradiating a target with a charged particle beam and comprising:
- a particle accelerator to generate the charged particle beam;
- an isocentric gantry rotatable about an axis Y, said gantry comprising a sequence of bending magnets arranged along a beam path and including a first bending magnet and a last bending magnet, the first bending magnet being configured to receive the particle beam along the axis Y and to bend and direct the particle beam away from the axis Y, the last bending magnet being configured to bend and direct the particle beam towards the isocentre;
- a first scanning magnet arranged along the beam path and configured to scan the particle beam over the target; and
- a magnetic resonance imaging (MRI) system comprising two separate main magnet units arranged respectively on opposite sides of the isocentre, said two main magnet units being configured to generate together a main magnetic field (Bo) which is parallel to or coincident with the axis Y.

The last bending magnet is configured to bend the particle beam in a first plane P1 comprising the isocentre and making an angle alpha with the axis Y such that alpha is larger than 70 degrees and smaller than or equal to 90 degrees. The first scanning magnet is arranged on the gantry between the said first bending magnet and the said last bending magnet and is configured to scan the particle beam in the first plane P1.

With such a configuration, the first scanning magnet can indeed be placed at a large distance (taken along the beam path) of the isocentre, yet without increasing the radius of the gantry, and, at the same time, a large field of scanning of the target can be achieved in at least the first plane P1, yet keeping a gap of the last bending magnet small and its poles large so as to keep its stray field low and hence reduce the influence of its magnetic field on the MRI system. Having a last bending magnet with a small gap and large poles allows also to achieve a good optical quality and to reduce its power consumption.

Preferably, the angle alpha is larger than 80 degrees and smaller than or equal to 90 degrees. More preferably, the angle alpha is equal to 90 degrees, which means that the first plane P1 is perpendicular to the axis Y. Increasing the angle alpha increases indeed the available field of scanning of the target in the first plane P1 and hence the size of the target which can be treated in this dimension.

Preferably, the apparatus further comprises a second scanning magnet arranged on the gantry between the said last bending magnet and the isocentre and configured to scan the particle beam in a second plane P2 , said second plane P2 comprising the axis Y. This allows to further scan the particle beam over the target according to another direction, without being concerned by constraints on the bending magnets of the gantry.

Preferably, the particle therapy apparatus further comprises a movable table to receive and transport the target to the isocentre, and a drive system to drive said movable table in translation along the axis Y while the target is in an irradiation position. This allows to move the target according to the direction of the axis Y and hence to scan the beam over parts of the target which could otherwise not be reached by scanning due to the small gap of the last bending magnet and/or due to the small free air gap between the two main magnet units of the MRI system. The first scanning magnet is in such a case preferably configured to scan the particle beam over the target and in the said first plane P1 only. This allows to reduce the cost, weight and bulk of the first scanning magnet compared to a case where this scanning magnet is configured to scan the beam according to two different (generally orthogonal) directions. Alternatively or additionally, the apparatus preferably further comprises a second scanning magnet arranged on the gantry between the said last bending magnet and the isocentre and configured to scan the particle beam in a second plane P2 comprising the axis Y. This allows to scan the particle beam over the target according to the Y direction which is transversal to the scanning direction offered by the first scanning magnet and without needing to increase the gap in the last bending magnet, even if the scanning range in this Y direction will be limited by the free air gap between the two main magnet units of the MRI.

Preferably, the particle accelerator is a cyclotron or a synchrotron.
Preferably, the particle beam is a beam of electrically charged particles excluding electrons, such as protons or carbon ions for example.

### Short description of the drawings

These and further aspects of the invention will be explained in greater detail by way of examples and with reference to the accompanying drawings in which:
- Fig.1 1: schematically shows a part of particle apparatus according to the invention;
- Fig.2: shows the apparatus of Fig.1 according to another view;
- Fig.3: schematically shows a part of a particle apparatus according to a preferred embodiment of the invention;

The drawings of the figures are neither drawn to scale nor proportioned. Generally, similar or identical components are denoted by the same reference numerals in the figures.

### Detailed description of embodiments of the invention

A particle therapy apparatus (1) for irradiating a target (5) with a charged particle beam (2) for therapy purposes is generally widely known form the prior art and comprises a particle accelerator to generate the charged particle beam (2), a beam transport system to transport the particle beam (2) from the particle accelerator to the target (5) to be irradiated, and various other subsystems, for example to shape the beam and/or to modify its energy and/or its intensity for the particular therapy envisaged. The target (5) may for example be a diseased part such as a tumor in a patient's body. Hence, the term "target (5)" used hereinafter may refer as well to such a tumor as to the patient himself.

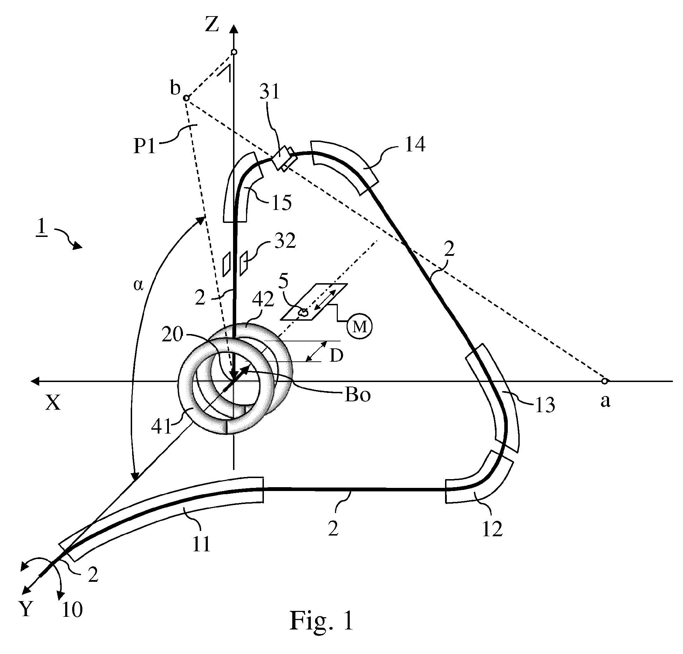

Fig.1 schematically shows a part of a particle therapy apparatus (1) according to an exemplary embodiment of the invention.
It comprises a particle accelerator (not shown for the sake of clarity) to generate the charged particle beam (2), an isocentric gantry (10) which is rotatable about an axis Y, and a part of a beam transport system (also not shown for the sake of clarity) to transport the particle beam (2), from where it is extracted from the particle accelerator to an entry point of the particle beam (2) into the gantry (10). In the present case, the particle beam (2) enters the gantry (10) beam line in parallel to or coincident with the rotation axis Y of the gantry (10). Fig. 1 also shows an orthogonal XYZ referential whose origin coincides with the isocentre (20) of the gantry (10) and whose Y axis is the axis of rotation of the gantry (10). The rotatable gantry (10) comprises and supports a plurality of bending magnets arranged in sequence along a beam path and including a first bending magnet (11) and a last bending magnet (15). Again for clarity reasons, a structure of the gantry (10) which is supporting the bending magnets is not shown on the figures. The first bending magnet (11) of said sequence is configured to receive the particle beam (2) along the axis Y and to bend and direct the particle beam (2) away from the axis Y, as shown on Fig.1. The last bending magnet (15) of said sequence is configured to bend and direct the particle beam (2) towards the isocentre (20), as also shown on Fig.1. The said sequence may comprise one or more additional bending magnets arranged on the gantry (10) along the beam path between the first bending magnet (11) and the last bending magnet (15).
The rotatable gantry (10) is said to be isocentric because the particle beam (2)s exiting from the last bending magnet (15) for any angle of rotation of the gantry (10) will all cross at a same point called the "isocentre (20)", hereinafter sometimes referred to as the isocentre (20) of the gantry (10). In practice, and as is well known, due to the heavy weight and mechanical imperfections of the system, the isocentre is strictly speaking not a single point but rather a small sphere.

Specific to the invention is that the last bending magnet (15) of the sequence is configured to bend the particle beam (2) in a first plane P1 which comprises the isocentre (20) and which makes an angle alpha with the axis Y such that alpha is larger than 70 degrees and smaller than or equal to 90 degrees. Preferably, the angle alpha between the first plane P1 and the axis Y is larger than 80 degrees and smaller than or equal to 90 degrees.
It is to be noted that that alpha is the angle between the axis Y and an orthogonal projection of Y on the said first plane P1. Hence, alpha will be equal to 90° in case the first plane P1 is perpendicular to Y.

In the example of Fig.1, the gantry (10) comprises a sequence of five beam bending magnets: a first (11), a second (12), a third (13), a fourth (14), and a fifth and last (15) bending magnet. The first bending magnet (11) is configured to receive the particle beam (2) along the Y axis, to bend the beam by 90° in the XY plane. The fifth and last bending magnet (15) is configured to bend the particle beam (2) in a first plane P1 making an angle with alpha with the Y axis which is larger than 70 degrees and smaller than or equal to 90 degrees, and to direct the particle beam (2) towards the axis Y at an isocentre (20) of the gantry (10). On Fig.1, the first plane P1 is the plane formed by the following three points: the isocentre (20), point "a" and point "b". The second, third, and fourth bending magnets are together configured to bend the particle beam (2) from an exit of the first bending magnet (11) to an entry into the fifth and last bending magnet (15). Such a gantry (10) is known for example from patent publication EP0635849 and is sometimes referred to as a supertwist gantry (10).
Other sequences and configurations of bending magnets may of course be used, provided the last bending magnet (15) is configured to bend the particle beam (2) in the first plane P1.

The particle therapy apparatus (1) further comprises a first scanning magnet (31) arranged along the beam path and configured to scan the particle beam (2) over the target (5) when the target (5) is placed at the isocentre (20) of the gantry (10). Such a scanning magnet is known in the art. Specific to the invention is that the first scanning magnet (31) is arranged on the gantry (10) between the said first bending magnet (11) and the said last bending magnet (15), and that the first scanning magnet (31) is configured to scan the particle beam (2) in the first plane P1. In other words, the first scanning magnet (31) is configured in such a way that, when activated, the particle beam (2) will move angularly in the first plane P1. Hence, one can use a last bending magnet (15) with a small gap between its poles so as to reduce its stray field.

The particle therapy apparatus (1) further comprises a magnetic resonance imaging (MRI) system comprising two separate main magnet units configured to generate together a main magnetic field (Bo), as are known in the art of MRI system s. Specific to the invention is that the two main magnet units (41, 42) are disposed respectively on opposite sides of the isocentre (20) and that they are arranged and configured to generate together a main magnetic field (Bo) which is parallel to or coincident with the axis Y. As shown on Fig.1, a first main magnet unit (41) is arranged on one side of the isocentre (20) and a second main magnet unit (42) is arranged on the opposite side of the isocentre (20). In this example, the first and second main magnet unit (42)s are arranged in such a way that the main magnetic field (Bo) which they generate upon their excitation is coincident with the axis Y at the isocentre (20). A bore in each of the first and second main magnet units (41, 42) allows to introduce a patient within the MRI and to place the target (5) at the isocentre (20).
The two main magnet units (41, 42) are separated from each other by a free air gap having a width D in the Y direction. In some examples, the width D is smaller than 15 cm and larger than 1 cm. In other examples, the width D is smaller than 10 cm and larger than 1 cm. In other examples, the width D is smaller than 8 cm and larger than 1 cm.
Hence, with the aforementioned arrangement of the gantry (10) and of the first scanning magnet (31), the particle beam (2) can be scanned over the target (5) in the X direction or in a direction close to the X direction, by passing through the said free air gap of the MRI.

Fig.2 shows the apparatus of Fig.1 according to an YZ plan view. In this view, one can better see the angle alpha (α) between the first plane P1 and the axis Y. In this example, the first plane P1 is perpendicular to the YZ plane, but other orientations are of course possible, provided the plane P1 comprises the isocentre and provided the angle alpha (α) is larger than 70 degrees and smaller or equal to 90 degrees. One can also better see that alpha has to be large enough in order to allow the particle beam (2) to reach the isocentre (20) without being hindered by the two main magnet units (41, 42) of the MRI.

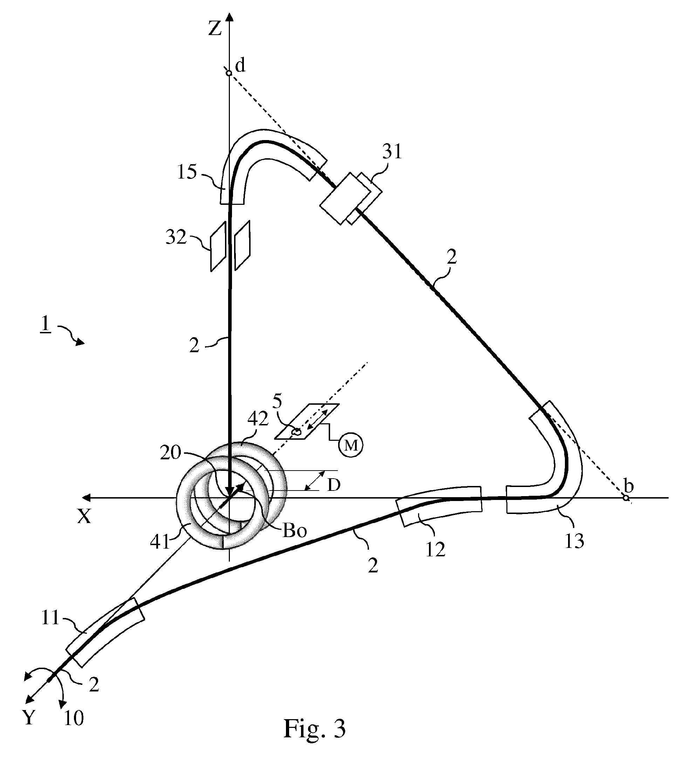

Fig.3 schematically shows a part of an exemplary particle apparatus according to a preferred embodiment of the invention. It is similar to the example of Fig.1 except that, in this case, the angle alpha between the first plane P1 and the axis Y is equal to 90 degrees. Fig. 3 also shows an orthogonal XYZ referential whose origin coincides with the isocentre (20) of the gantry (10) and whose Y axis is the axis of rotation of the gantry (10). In such referential, the plane P1 is the XZ plane.
In this example, the gantry (10) comprises a sequence of four beam bending magnets: a first (11), a second (12), a third (13), and a fourth (last) (15) bending magnet. The first bending magnet (11) is configured to receive the particle beam (2) along the Y axis, to bend the beam by 45° in the XY plane, and to direct the beam towards the second bending magnet (12). The second bending magnet (12) is configured to bend the beam by 45° in the XY plane, and to direct the beam along the X axis and towards the third bending magnet (13). The third bending magnet (13) is configured to bend the beam by 135° in the XZ plane, and to direct the beam back towards the YZ plane and towards the fourth (last) bending magnet (15). The fourth (last) bending magnet (15) is configured to bend the beam by 135° in the first plane P1 (XZ plane), and to direct the beam towards the isocentre (20) of the gantry (10). Such a gantry (10) is sometimes referred to as a corkscrew gantry (10) and an example thereof is described in patent publication number WO8909906.
Other configurations of the bending magnets may of course be used, provided the last bending magnet (15) is configured to bend the particle beam (2) in the first plane P1, which in this example is the XZ plane.
As with the example of Fig.1, the particle therapy apparatus (1) further comprises a first scanning magnet (31) arranged along the beam path between the said first bending magnet (11) and the said last bending magnet (15) and configured to scan the particle beam (2) in the first plane P1 and over the target (5) when the target (5) is placed at or at proximity of the isocentre (20) of the gantry (10).
As an advantageous consequence thereof, one can use a last bending magnet (15) with a small gap between its pole faces so as to reduce its stray field.
The particle therapy apparatus (1) further comprises a magnetic resonance imaging (MRI) system comprising two separate main magnet units arranged and configured as described in relation to Fig.1.

In an apparatus according to the invention, the first scanning magnet (31) is preferably configured to scan the particle beam (2) in the first plane P1 only. Preferably, the first scanning magnet (31) is arranged between a one before last and the last bending magnet (15) of the said sequence of bending magnets of the gantry (10).

The apparatus according to the invention preferably further comprises a second scanning magnet (32) which is arranged on the gantry (10) between the said last bending magnet (15) and the isocentre (20) of the gantry (10). Said second scanning magnet (32) is configured to scan the particle beam (2) in a second plane P2, said second plane P2 comprising the axis Y. In other words, the second scanning magnet (32) is configured in such a way that, when activated, the particle beam (2) will move angularly in the second plane P2. In the examples of Fig.1 and Fig.3, the second scanning magnet (32) is configured to move the particle beam (2) in the YZ plane so that the target (5) can also be scanned in the Y direction. Since the particle beam (2) still has to pass through the free air gap between the two main magnet units (41, 42) of the MRI, the scanning range of the second scanning magnet (32) will be smaller than the scanning range of the first scanning magnet (31). Therefore, the second scanning magnet (32) will preferably be smaller and have a lower maximum power than the first scanning magnet (31).

The apparatus according to the invention preferably further comprises a movable table (50) which is adapted to receive and transport the target (5) (for example the patient) to the isocentre (20), and a drive system (51) to drive said movable table (50) in translation along the axis Y while the target (5) is in an irradiation position. Such a device are generally known in the art and is sometimes referred to as a patient robot or a robotic patient positioner. Specific to this preferred embodiment is that the drive system (51) is configured to move the target (5) (the patient) according to the Y direction while the target (5) (the patient) is in an irradiation position. The combination of scanning the particle beam (2) with the first scanning magnet (31) and moving the target (5) along the axis Y with the drive system (51) makes it possible to proceed for example with raster scanning of the target (5) according to two different (generally orthogonal) directions. In such a case, the second scanning magnet (32) may or may not be present in the apparatus (1).

Preferably each of the two main magnet units (41, 42) of the magnetic resonance imaging system comprises a superconducting electromagnet. As is known in the art, each of the two main magnet units comprises in this case a coil enclosed in a cryostat having external walls. The aforementioned free air gap between the two main magnet units (41, 42) is then of course the gap between the external walls of the two main magnet units (41, 42) and the width (D) of this free air gap is the free distance between the two facing external walls of the two main magnet units (41, 42), as shown on the figures.

Preferably, the first main magnet unit (41) and second main magnet unit (42) of the MRI system are arranged symmetrically with respect to the isocentre (20) of the gantry (10), so that an imaging center of the MRI will substantially coincide with the isocentre (20) of the gantry (10).

Possible influence of the main magnetic field Bo of the MRI system on the beam path, for example in the vicinity of the two main magnet units (41, 42) of the MRI system, may be corrected by acting on the excitation current of at least one of the bending magnets of the gantry (10) - preferably on the excitation current of the last bending magnet (15) - and/or on the excitation current of the first and/or second scanning magnet (32), and/or by any other known means which are capable of modifying the path of the particle beam (2), such as steering magnets for instance.

Preferably, the particle beam (2) is a beam of electrically charged particles, excluding electrons. More preferably, the particle beam (2) is a beam of protons or a beam of carbon ions. Preferably, the particle accelerator is a cyclotron or a synchrotron, more preferably a synchrocyclotron, even more preferably a superconducting synchrocyclotron. Preferably, the particle accelerator is adapted to generate and deliver a beam of charged particles whose energy is higher than 60 MeV.

The present invention has been described in terms of specific embodiments, which are illustrative of the invention and not to be construed as limiting. More generally, it will be appreciated by persons skilled in the art that the present invention is not limited by what has been particularly shown and/or described hereinabove.
Reference numerals in the claims do not limit their protective scope.
Use of the verbs "to comprise", "to include", "to be composed of", or any other variant, as well as their respective conjugations, does not exclude the presence of elements other than those stated.
Use of the article "a", "an" or "the" preceding an element does not exclude the presence of a plurality of such elements.

The invention may also be described as follows: a particle therapy apparatus (1) for irradiating a target (5) with a charged particle beam (2) and comprising a particle accelerator and an isocentric gantry (10) rotatable about an axis Y. The gantry (10) comprises a sequence of bending magnets to successively bend and finally direct the particle beam (2) towards the isocentre (20) of the gantry (10). A last bending magnet (15) of said sequence is configured to bend the particle beam (2) in a first plane P1 comprising the isocentre (20) and making a large angle (preferably more than 70 degrees, more preferably more than 80 degrees, even more preferably equal to 90 degrees) with the axis Y. The gantry (10) also comprises a first scanning magnet (31) arranged upstream of the last bending magnet (15) and configured to scan the particle beam (2) over the target (5) in the first plane P1. The apparatus also comprises a magnetic resonance imaging (MRI) system having two main magnet units (41, 42) arranged respectively on opposite sides of the isocentre (20) and configured to generate together a main magnetic field (Bo) which is parallel to or coaxial with the axis Y. MRI imaging of the target (5) at the isocentre (20) can hence be achieved while or while not scanning the particle beam (2) over the target (5).

## Claims

1. A particle therapy apparatus (1) for irradiating a target (5) with a charged particle beam (2) and comprising:
- a particle accelerator to generate the charged particle beam (2);
- an isocentric gantry (10) rotatable about an axis Y, said gantry (10) comprising a sequence of bending magnets arranged along a beam path and including a first bending magnet (11) and a last bending magnet (15), the first bending magnet (11) being configured to receive the particle beam (2) along the axis Y and to bend and direct the particle beam (2) away from the axis Y, the last bending magnet (15) being configured to bend and direct the particle beam (2) towards the isocentre (20);
- a first scanning magnet (31) arranged along the beam path and configured to scan the particle beam (2) over the target (5); and
- a magnetic resonance imaging (MRI) system comprising two separate main magnet units (41, 42) arranged respectively on opposite sides of the isocentre (20), said two main magnet units (41, 42) being configured to generate together a main magnetic field (Bo) which is parallel to or coincident with the axis Y,
**characterized in that**
the last bending magnet (15) is configured to bend the particle beam (2) in a first plane P1 comprising the isocentre (20) and making an angle alpha with the axis Y such that alpha is larger than 70 degrees and smaller than or equal to 90 degrees,
and **in that**
the first scanning magnet (31) is arranged on the gantry (10) between the said first bending magnet (11) and the said last bending magnet (15) and is configured to scan the particle beam (2) in the first plane P1.

2. A particle therapy apparatus (1) according to claim 1, **characterized in that** the angle alpha is larger than 80 degrees and smaller than or equal to 90 degrees.

3. A particle therapy apparatus (1) according to claim 2, **characterized in that** the angle alpha is equal to 90 degrees.

4. A particle therapy apparatus (1) according to any of preceding claims, **characterized in that** the apparatus further comprises a second scanning magnet (32) arranged on the gantry (10) between the said last bending magnet (15) and the isocentre (20) and configured to scan the particle beam (2) in a second plane P2 , said second plane P2 comprising the axis Y.

5. A particle therapy apparatus (1) according to any of preceding claims, **characterized in that** the apparatus further comprises a movable table (50) to receive and transport the target (5) to the isocentre (20), and a drive system (51) to drive said movable table (50) in translation along the axis Y while the target (5) is in an irradiation position.

6. A particle therapy apparatus (1) according to any of preceding claims, **characterized in that** the first scanning magnet (31) is configured to scan the particle beam (2) in the first plane P1 only.

7. A particle therapy apparatus (1) according to any of preceding claims, **characterized in that** the first scanning magnet (31) is arranged between a one before last and the last bending magnet (15) of the said sequence of bending magnets.

8. A particle therapy apparatus (1) according to any of preceding claims, **characterized in that** a width (D) of a free air gap between the two main magnet units (41, 42) of the magnetic resonance imaging system is smaller than 15 cm.

9. A particle therapy apparatus (1) according to claim 8, **characterized in that** the width (D) of the free air gap between the two main magnet units (41, 42) of the magnetic resonance imaging system is smaller than 10 cm.

10. A particle therapy apparatus (1) according to any of preceding claims, **characterized in that** each of the two main magnet units (41, 42) of the magnetic resonance imaging system comprises a superconducting electromagnet.

11. A particle therapy apparatus (1) according to any of preceding claims, **characterized in that** the said isocentre (20) of the gantry (10) coincides with an imaging centre of the magnetic resonance imaging system.

12. A particle therapy apparatus (1) according to any of preceding claims, **characterized in that** the particle beam (2) is a beam of electrically charged particles, excluding electrons.

13. A particle therapy apparatus (1) according to claim 10, **characterized in that** the particle beam (2) is a beam of protons or a beam of carbon ions.

14. A particle therapy apparatus (1) according to any of preceding claims, **characterized in that** the particle accelerator is a cyclotron or a synchrotron.
